# EUROPEAN PATENT SPECIFICATION

(11) **EP 1 772 114 B1**
(45) Date of publication and mention of the grant of the patent: **28.09.2011**
(21) Application number: 06015527.2
(22) Date of filing: 05.06.2001
(51) Int. Cl.: A61F 2/06

(54) **Intravascular stent with increasing coating retaining capacity**
Intravaskulärer Stent mit verbessertem Haltevermögen einer Beschichtung
Extenseur intravasculaire doté d'une capacité de rétention d'enrobage améliorée

(30) Priority: 05.06.2000 US 209255 P
(43) Date of publication of application: 11.04.2007
(62) Divisional of application: 01939926.0
(73) Proprietor: Boston Scientific Limited, St. Michael, Barbados, West Indies (BB)
(72) Inventor: Jang, David G., Redlands, CA 92374 (US)
(74) Representative: Vossius & Partner

(56) References cited:
- WO-A-00/30563
- WO-A-98/36784

## Description

### BACKGROUND OF THE INVENTION

### Field of Invention:

This invention relates generally to intravascular stents, and more particularly to intravascular stents that include a plurality of cavities formed on a surface of the stent and are coated with a restenosis inhibiting agent

### Description of the Related Art:

By 1999, the percutaneous balloon angioplasty and stent implant procedures have become the dominant non-surgical revascularization method of the atherosclerotic stenosis, or obstruction, of the vascular lumen, and particularly in the coronary vascular system in the heart. With balloon angioplasty alone, without use of stent, the restenosis rate after angioplasty has been as high as 25-45% in the first time clinical cases. With use of stents after balloon angioplasty, the restenosis has been reduced significantly. Even so, the restenosis rate after stent implant is reported as 10-25% range depending on the condition of the vessel stented or what specific stent was used, requiring a need for further restenosis reducing measures after intravascular stenting.

To further reduce the restenosis rate after stent implant, numerous means has been tried, including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient and costly. Mainly because it is radioactive device and radiation therapy specialist from another department has to be involved with the interventional cardiologist in the cardiac catheterization laboratory. The laser and atherectomy devices proved to be marginally useful in this purpose with added costs.

The local drug therapy appears be a very promising method for the future, as better pharmaceutical, chemical or biogenetic agents are developed and became available. Some research data, both from animal tests and human clinical studies, indicate that there are evidences of suppressing restenosis after stent implant when certain growth blocking pharmaceutical agents available today are used to coat the stent. In another instances, it has been speculated that certain surface modifying materials coated on the surface of the stent may be beneficial by it alone or in combination with growth suppressing agent, in reducing restenosis rate. In either instance, the drug or substance should be locally attached or coated on the stent and in sufficient amounts. However, attaching or coating a sufficient amount of a substance or drug on the coronary stent is not so easy a proposition.

Coating a drug or an agent on the surface of the stent has a demanding problem of enough volume of such substance coated on the small surface areas of stent struts, without increasing the physical width or thickness of stent struts. This demand directly conflicts with the metal fraction issue of the stent If the width (and lesser degree the thickness) of stent struts is increased in order to widen drug coating surface areas, it would have an elevated deleterious foreign body effect of the increased metal fraction of the stent, which would promote restenosis.

Designing an ideal stent, particularly the coronary stent, is a very demanding balance of a numerous conflicting factors. An ideal stent requires an ideal balance of numerous different stent features built into the stent. One of the many requirements of a coronary, or any vascular stent, is to keep the metal fraction of the stent low. This means that drug coating is a very demanding task. Enough amounts of a drug or agent should be coated on the miniscule surface areas of the stent struts, in order to have the desired drug results of reducing restenosis. An average stent, particularly a coronary stent, will have problem of providing desired amount of drug-retaining capacity on the surface areas of the stent struts.

The main invention of this application is not an invention of the stent itself. The present invention is the particular measures designed to increase drug coating or attachment capacity of a stent by adding exposed surface areas or reservoir capacity of the stent, without increasing the width or thickness of the stent struts or without increasing the metal fraction of the stent. These special measures of present invention will enhance the coating substances to a stent. Further, the present invention will enhance the reservoir capacity of the stent for different forms of restenosis reducing proteins, chemicals or drugs, and will prolong the releasing time duration of the substances.

U.S. Patent No. 6,190,404 discloses an intravascular stent with an outer surface, an inner surface and grooves formed in the inner surface of the stent. The grooves are positioned and provided to increase the rate of migration of endothelial cells upon the inner surface of the stent.

There is a need for a stent with a geometry that provides for an increased amount of a coating substance. There is a further need for a stent that includes reservoirs for retaining coatings.

From WO 00/30563 a segmented articulatable stent of open structure has become know, comprising end-connected expansion struts of first and second lengths making up first and second segments with angular connector struts interconnecting adjacent first and second segments.

From WO98/36784 a coated implantable medical device has become known, preferably a coronary stent. In one embodiment the stent structure includes apertures provided as holes or wells for containing a bioactive material.

WO 97/40781 relates to a stent assembly including a balloon and an expandable stent position at an exterior of the balloon. The stent includes a first expansion strut. A plurality of first expansion struts form a first expansion column. A plurality of second expansion struts form a second expansion column. A plurality of the first connecting struts form a first connecting strut column. Each of the first expansion strut of the first expansion column is coupled to a second expansion strut of the second expansion column. The first connecting strut includes a proximal section coupled to a distal section and at least a portion of the first connecting strut includes a first linear section and a first slant angle.

WO 99/23977 discloses an intravascular stent having an outer surface and an inner surface, wherein the inner surface is provided with at least one groove which allegedly increases the rate of migration of endothelial cells upon the inner surface of the stent after it has been implanted.

### SUMMARY OF THE INVENTION

Accordingly, an object of the present invention is to provide an intravascular stent with a geometry that provides for an increased amount of a coating substance. Another object of the present invention is to provide an intravascular stent with cavities formed in the stent that serve as reservoirs of coatings applied to the stent. Yet another object of the present invention is to provide an intravascular stent with cavities formed in the body of the stent and with a restenosis inhibiting agent applied to the stent.

Another object of the present invention is to provide an intravascular stent with micro-holes or micro-slits that provide reservoirs for stent coatings.

These and other objects of the present invention are achieved with the expandable stent, as recited in claim 1. Generally, the expandable stent comprises a tubular structure including an outer surface positionable adjacent to a vessel wall and an inner surface facing a lumen of a body passageway. The tubular structure further includes a plurality of expansion struts, connector struts and cells. The tubular structure has a first diameter which permits intraluminal delivery of the tubular structure into the body passageway, and a second expanded and deformed diameter which is achieved upon the application of a radially, outwardly extending force. A plurality of cavities are formed in the outer surface of the stent.

In an embodiment of the present invention, a coating substance is provided on at least a portion of outer surface of the stent including and extending into at least a portion of the cavities.

In another embodiment of the present invention, a stent assembly includes a balloon and the expandable stent positioned at an exterior of the balloon, wherein the second expanded and deformed diameter is achieved upon the application of a radially, outwardly extending force applied by the balloon.

In another embodiment of the present invention, a method of manufacturing an intravascular stent is provided as recided in claim 45. The intravascular stent has an inner surface and an outer surface. A plurality of cavities are formed on the outer surface. A coating substance that inhibits restenosis may be formed on at least a portion of the outer surface and on at least a portion of the plurality of cavities.

### BFIEF DESCRIPTION OF DRAWINGS

Figure 1 is a flat cut-open, two-dimensional, schematic view of one embodiment of a stent of the present invention that includes cavities formed in the body of the stent.
Figure 2 is a close-up view of the stent from Figure 1 with cavities that extend from the outer surface of the stent into an interior of the stent.
Figure 3 is a cross-section, side view of a stent of the present invention with cavities that extend from the outer surface through the inner surface.
Figure 3(b) is a cross-sectional, magnified, side view of one embodiment of the stent of the present invention illustrating that cavities can be closed and serve as reservoirs for coating substance applied to the stent..
Figure 3(c) is a cross-sectional, magnified view of another embodiment of the present invention illustrating a stent that includes cavities that extent at a slant angle from the outer surface through the inner surface.
Figure 3(d) is a cross-sectional, magnified, side view of one embodiment of the present invention with cavities that extend at a slant, non-perpendicular angle from the outer surface to an interior of the stent.
Figure 4 is a flat cut-open, two-dimensional, schematic view of a stent seen from the outer surface of the stent cavities distributed in an even pattern
Figure 5 is a close-up, magnified, view of the expansion and connector struts from Figure 4 with micro-slits and micro-holes that extend from the outer surface of the stent struts.
Figure 6(a) is a cross-sectional, magnified, side view of a stent strut of the present invention illustrating micro slits that extend through both the outer and inner surfaces and the entire thickness of the stent strut.
Figure 6(b) is a cross-sectional, magnified, side view of the stent strut of the present invention illustrating perpendicularly expending open micro slits on one side and closed micro-slits on the opposite site of the strut.

### DETAILED DESCRIPTION OF THE PREFERRED EMBODIMENTS

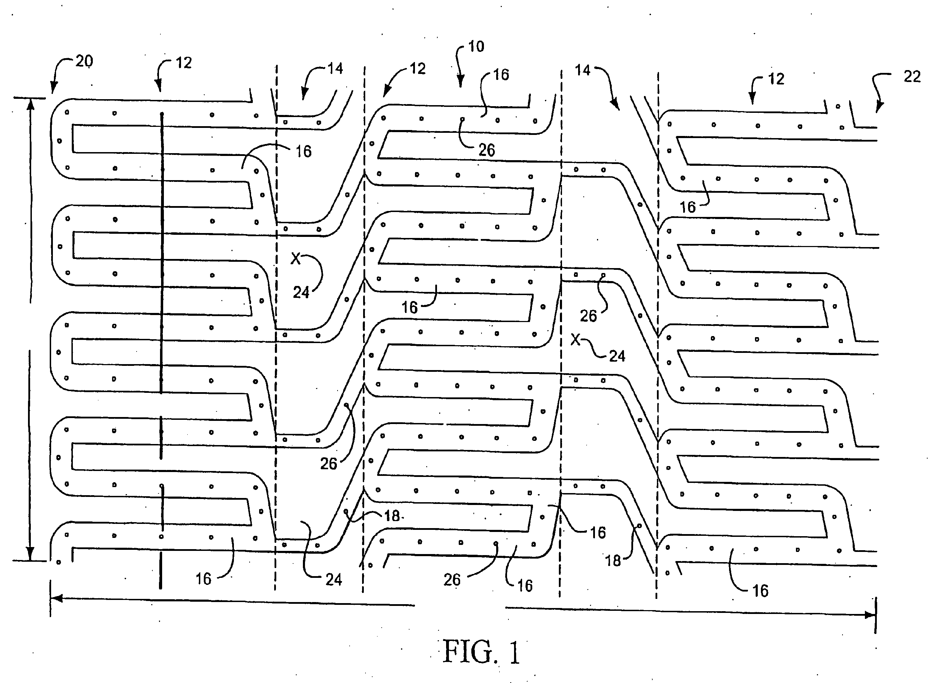

Referring now to Figure 1, one embodiment of an expandable stent 10 of the present invention is illustrated. A tubular structure includes an outer surface positionable adjacent to a vessel wall and an inner surface facing a lumen of a body passageway. The tubular structure further includes a plurality of expansion struts, connector struts and cells. The tubular structure has a first diameter which permits intraluminal delivery of the tubular structure into the body passageway, and a second expanded and deformed diameter which is achieved upon the application of a radially, outwardly extending force.

A plurality of cavities are formed in the outer surface of the stent. The cavities can be micro-holes or micro-slits and extend from the outer surface to an interior of the struts, or extend from the outer surface all the way through the inner surface. An example of a stent design useful with the present invention is disclosed in US Patent 5,954,743. In Figure 1, a two-dimensional view of the stent 10 is illustrated and is seen from the outer surface of the cut-open, two-dimensional view.

Stent 10 includes expansion columns 12 and connector columns 14 in a continuous and alternating pattern to form a longitudinal dimension and a vertical dimension. The vertical and longitudinal dimensions determine the circumference and the length respectively of stent 10. Expansion columns 12 have expansion struts 16 in a vertical zigzag or corrugated pattern. One expansion column 12 is linked to an adjacent expansion column 12 by connector column 14 between two adjacent expansion 12 columns. Connector columns 14 have connector struts 18 that serve as linking arms between expansion struts 16 in two adjacent expansion columns 12. Stent 10 has a proximal end 20 and a truncated end 22 in the middle of stent 10.

In one embodiment, stent 10 is a tubular structure that includes patterned expansion struts 16 and connectors struts 18 continuously linked circumferentially and longitudinally with a predetermined length. The total surface areas of struts 16 and 18 are limited to a certain percent of the total cylindrical surface area of tubular stent 10, particularly when stent 10 is expanded in a vessel, with enlarged (by stent expansion) stent cells 24 that make up the remainder of the total stent surface area.

The amount of a coating substance applied to and retained by stent 10 is determined by the total surface area of stent struts 16 and 18. Coating substance is preferably a restenosis inhibiting agent that is a drug, polymer and bio-engineered material and combinations thereof. It will be appreciated that other types of coating substances, well known to those skilled in the art, can be applied to stent 10 of the present invention. Because the total stent strut surface areas are limited in size, the amount of coating substance applied to stent 10 is limited to a small volume. When stent 10 is expanded in a vessel the relative surface area of struts 16 and 18 decreases in relation to the areas of stent cells 24. The total cylindrical surface area of stent 10 when it is implanted and expanded inside of a vessel is equal to the sum of the strut surface areas, which do not change, and stent cells 24 areas. The size of stent cells 24 areas changes when stent 10 is expanded. The present invention increases the amount of the coating substance capacity of stent 10 without increase the metal fraction of stent 10.

In various embodiments, the present invention increases the coating substance retaining capacity of stent 10 by forming cavities that can be micro holes 26 which are made, punched, drilled or burned into the expansion and connector struts 16. In Figure 1, micro holes 26 have openings 28 on outer surface of struts 16 and 18. Micro holes 26 are made and arranged in such a way so that they can be evenly distributed in struts 16 and 18. In this embodiment, micro holes 26 are evenly distributed through out the entire body of stent 10. The number of micro holes 26 illustrated in Figure-1 is only by example.

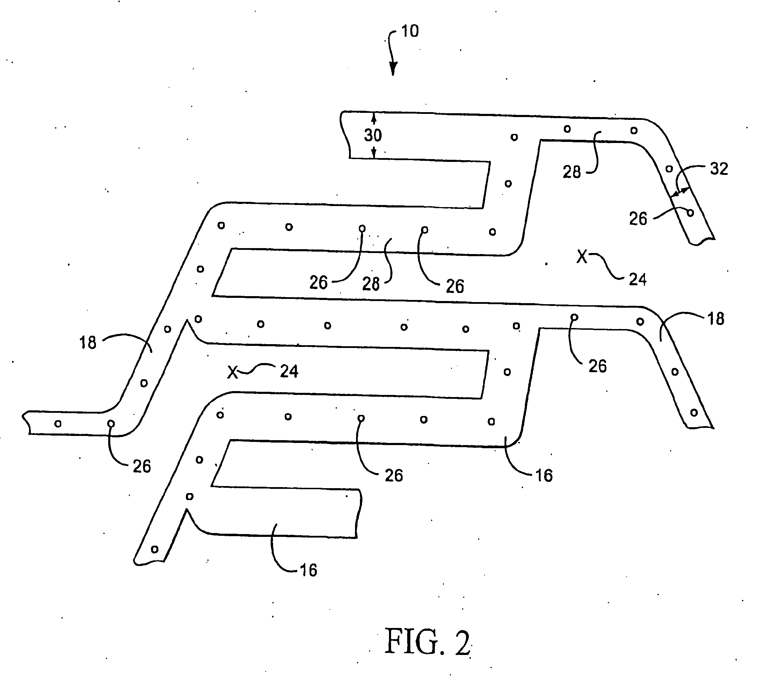

The number of micro holes 26 created in stent 10 can vary by increasing or decreasing the number according to the necessity and requirements when such stents are fabricated for clinical use. Additionally, the pattern of creating micro holes 26 in stent struts 16 and 18 can be varied according to the clinical and protocol needs. Although micro holes 26 in Figure 1 are made in straight lines it will be appreciated that micro holes 26 can be made in any varied pattern or shape. Micro-holes 26 can be made to form any suitable shape or pattern as necessary, if they meet the structural or engineering requirements of stent 10. Micro holes 26 can be made in single line or in multiple lines in struts 16 and 18 and arranged in any pattern. In the embodiment illustrated in Figure 2, width 30 of expansion strut 16 is shown as being larger than width 32 of connector strut 18.

The size of micro holes 26 can be based on the physical dimensions of struts 16 and 18. Micro holes 26 cannot have diameters or size as large as the width of struts 16 and 18. Micro holes 26 can have substantially smaller widths or diameters than widths 30 and 32 in order to maintain the structural integrity and radial strength of stent 10. In one embodiment, micro holes 26 have an effective size or diameter to provide an optimal retaining capacity of substances or drugs that are coated or deposited on stent 10. Similarly, the distance between micro holes 26 is selected to maintain the integrity of stent 10 while providing an optimal number of micro holes 26 to provide a sufficient coating substance retaining capacity. Micro holes 26 in struts 18 can be made smaller than micro holes 26 in expansion struts 16 and visa versa.

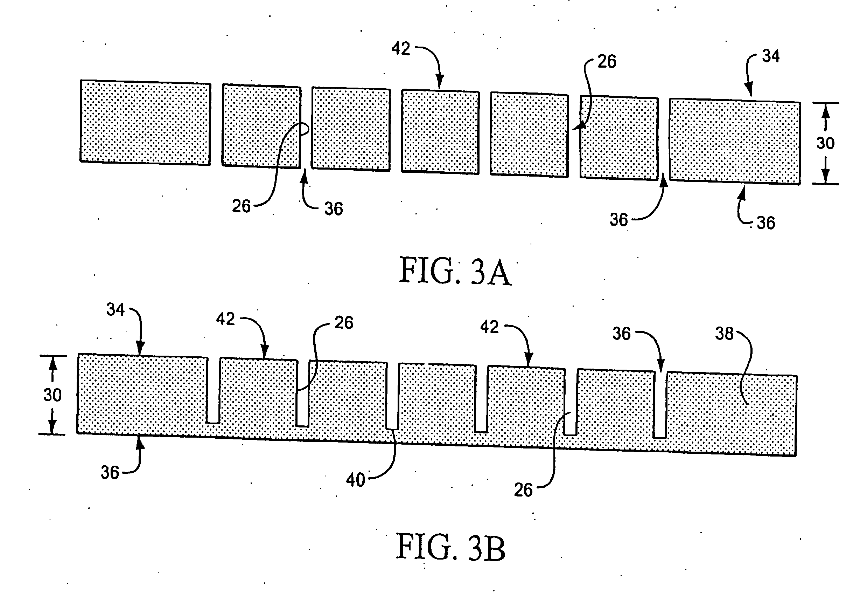

Micro holes 26 and opening 28 can have more than one shape including but not limited to circular, square, oval, oblong, irregular, polygonal or a combination thereof, depending on the method used to create micro holes 26. The tools to create micro holes 26 can be mechanical, photochemical, laser, EDM and the like.. The shape or configuration of micro holes 26 and openings 28 in stent struts 16 and 18 can be influenced by the size or diameter of the micro hole 26 made, as well as by other manufacturing factors such as a laser beam size, photochemical resolution or EDM cathode and the like. In the embodiment of Figure 3 (a), micro holes 26 penetrate the entire widths 30 and 32 of struts 16 and 18 at a perpendicular angle with opening 28 on both outer surface 34 and inner surface 36. Shaded areas 38 show the cross-sectional cut surface of struts 16 and 18. Micro holes 26 are created in a regular interval with the uninterrupted segment 28 between micro holes 26. Micro holes 26 communicate freely between outer surface 34 and inner surface 36. As can be seen, micro holes 26 increase the contact surface areas of stent struts 16 or 18 for the purpose of increasing the capacity of retaining the intended coating substance added to stent 10. The bore space of micro holes 26 also serve as micro reservoir chambers for the substance to be added, attached or coated to stent 10. When stent 10 is electropolished, the shape or dimension of micro holes 26 can be slightly changed.

Figure 3(b) illustrates an embodiment where micro holes 36 are blind and extend from outer surface 34 but not do not continue to inner surface 36. Shaded areas 38 indicate cross-sectioned stent struts 16 and 18. In Figure 3(b), micro holes 26 have cul de sac geometry's 40 that terminate in an interior of struts 16 and 18. Cul-de-sacs 40 serve as reservoirs for coating substances applied to stent 10. Cul-de-sacs 40 can be created at regular or irregular intervals with uninterrupted segments 42 between that are formed between micro holes 26.

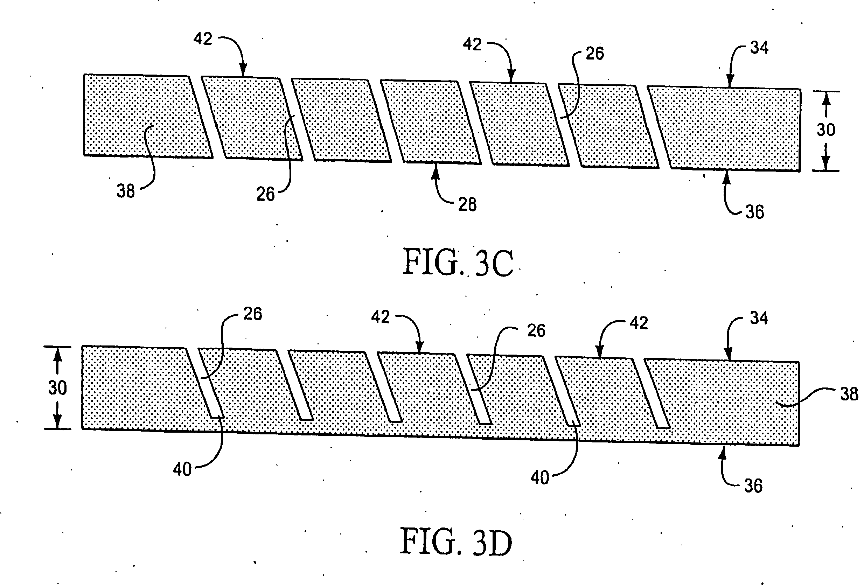

Referring now to Figure 3(c), micro holes 26 are shown with their axes at a slant angle relative to stent struts 16 and 18. In this embodiment, micro holes 26 extend from outer surface 34 to inner surface 36. Because micro holes 26 have a slant angle through in this embodiment, the length and reservoir capacity of the micro holes 26 is increased compared to the capacity of the Figure 3(a) micro holes 26.

In the embodiment illustrated in Figure 3(d), micro holes 26 have openings 28 on outer surface 34 and cul de sacs 40 on inner surface 36, all formed at a slant angle. Outer surface 34 has uninterrupted segments 42 between slant angled micro holes 26 and inner surface 36 is smooth without openings 28. Again, cul-de-sac 40 provides a reservoir for a coating substance applied to stent 10. Because the bore space of micro holes is at a slant angle there is an increased reservoir capacity.

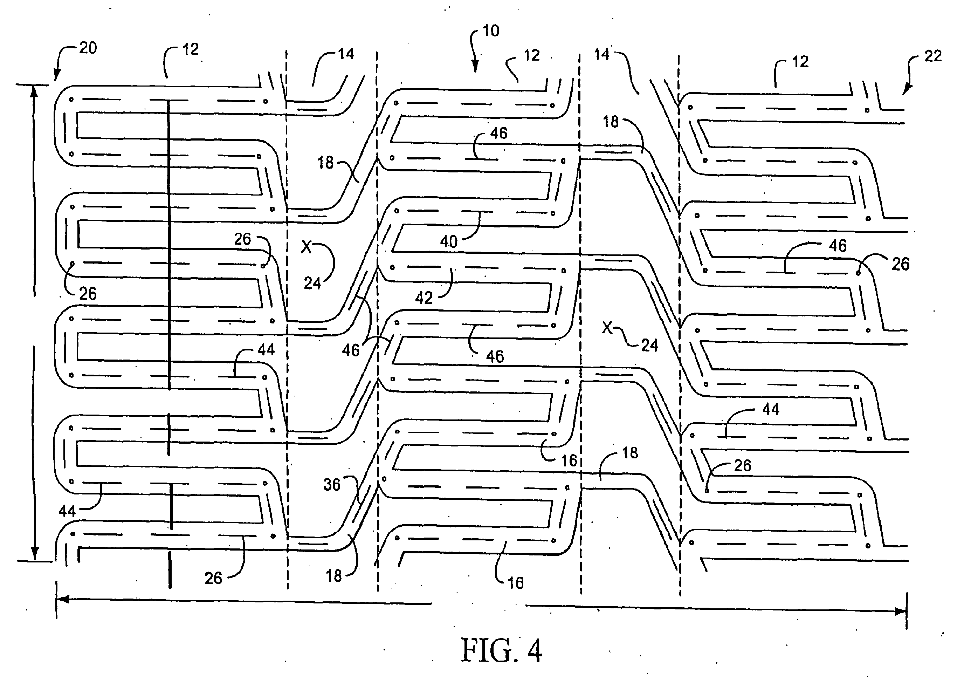

In contrast to Figure 1, the cavities formed in Figure 4 are micro slits, groves, and the like, collectively denoted as 40, which have widths 44 that are larger than openings 36. Compared to micro holes 26 of Figure 1, the micro slits 40 shown in Figure 4 provide a larger reservoir capacity for the coating substance.

Micro slits 40 can be evenly or unevenly distributed in struts 16 and 18.. The number of micro slits 40 illustrated in Figure-4 is only by way of example. Increasing or decreasing the number of micro slits 40 created in stent 10 may vary according to the clinical and pharmcodynamic necessity and requirements. Additionally, the pattern of creating micro slits 40 in struts 16 and 18 can vary according to the clinical and engineering needs. Although micro slits 40 illustrated in Figure-4 are in straight lines, micro slits 40 can be made in curvilinear, square-angles, slant angled and the like with or without radius of curvature. Micro slits 40 can be made in any suitable pattern or shape as required, if they meet the structural or engineering requirements of stent 10. Micro slits 40 can be made in single line or in multiple lines in struts 16 and 18 and arranged in any other pattern. The

Figure-4 embodiment illustrates that micro holes 26 can also be included in the same stent 10.

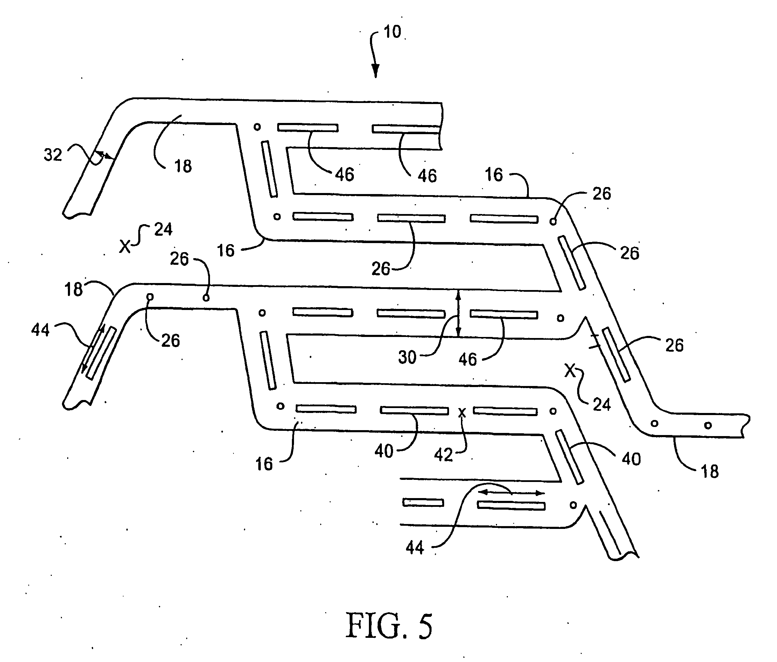

The magnified view of stent 10 illustrated in Figure 5 shows struts 16 and 18 with micro slits 40 and openings 46 on outer surface 34 of stent 10. Width 34 of expansion strut 16 is larger than width 32 of strut 18.

Width of micro slit 40 is determined by the physical dimensions and limits of struts 16 and 18. Width 44 can not be made as large as widths 34 and 32. Width is substantially smaller than width 34 in order to maintain the structural integrity and radial strength of stent 10. The length 44 of micro slit 40 can be made as long as stent struts 16 and 18. The length 44 of micro slit 40 can be shorter or longer than the width of struts 16 and 18. Similarly, the uninterrupted distance 42 between micro slits 40 is selected so that the structural integrity of stent 10 is not compromised. Within the allowable limits, micro slits 40 can be made in different sizes or dimensions in same or differing patterns. Micro slits 40 in struts 18 can be made smaller than, the same as or greater than micro-slits 40 formed in struts 16.

The shape of micro slits 40 and opening 46 can be different than that illustrated in Figure 5. The geometry of micro slits 40 can be straight linear, curvilinear, angled, squared or any other shape, depending on the design of stent 10 and the method used to make micro slits 40 during the manufacturing process. The tools to create the micro slits 40 can be the same as those used for micro-holes 26. Different shapes, sizes and positions of micro slits 40 can be included in an individual stent 10.

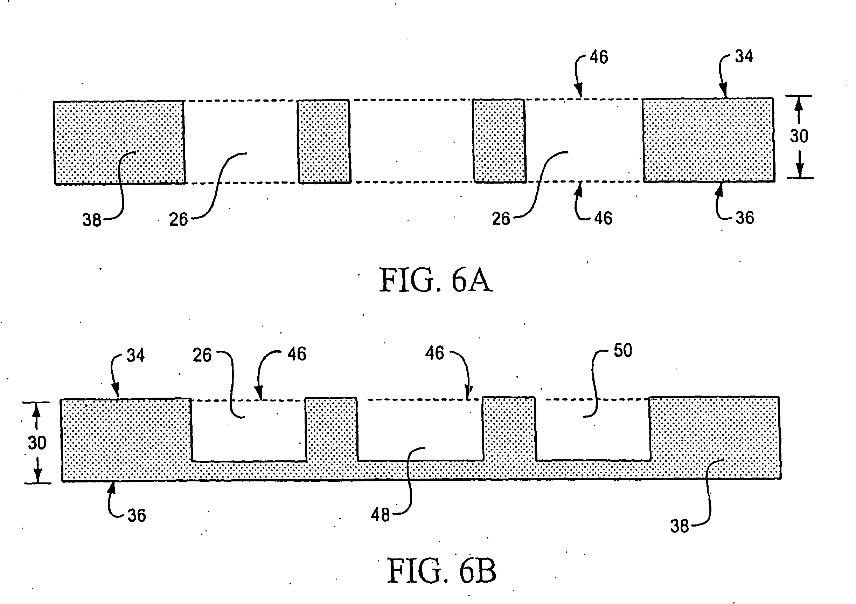

Referring now to Figure 6(a), micro slits 40 can extend through struts 16 and 18 with openings 50 on both outer and inner surfaces 34 and 36. Micro slits 40 can be created in a regular interval with uninterrupted segment 42 between micro slits 40.

Additionally, in this embodiment micro slits 40 can communicate freely between outer surface 34 inner surface 36. Micro slits 40 increase coating substance contact surface areas of struts 16 and 18 for the purpose of increasing the reservoir capacity of intended coating substances.

Figure-6(b) illustrates that blind micro slits 40 partially extend into struts 16 and 18 from outer surface 34. Blind micro slits 40 end in cul-de-sacs 48 which can be of any desired geometric configuration. Cul-de-sacs 48 create micro reservoirs 50 for coating substances and can be formed at regular or irregular intervals with uninterrupted segments 42 between blind micro slits 48. Generally, the reservoir capacity of blind micro slits 40 is greater than that of blind micro holes 26. Additionally, Micro slits 40 can also be made in slant angles.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. It is intended that the scope of the invention be defined by the following claims and their equivalents.

## Claims

1. An expandable stent (10), comprising:
a tubular structure including an outer surface positionable adjacent to a vessel wall, an inner surface facing a lumen of a body passageway, a plurality of expansion struts (16), connector struts (18) and cells (24), the tubular structure having a first diameter which permits intraluminal delivery of the tubular structure into the body passageway having a lumen, and a second diameter in the expanded shape of the stent upon the application from the interior of the tubular structure of a radially, outwardly extending force, the cells having areas which enlarge upon expansion of the stent, wherein the width (30) of the expansion struts (16) is larger than the width (32) of the connector struts (18), **characterized by**
a plurality of cavities (26, 40) formed in the stent providing as reservoirs of a coating substance, wherein the cavities extend from the outer surface of the stent through the inner surface of the stent, and wherein the cavities are oblong, square and/or oval.

2. The stent of claim 1, wherein the tubular structure is balloon expandable.

3. The stent of claim 1, wherein the tubular structure is self-expandable.

4. The stent of claim 1, wherein at least a portion of the tubular structure is made of a shape memory alloy.

5. The stent of claim 1, wherein the plurality of cavities (26, 40) are substantially evenly positioned on the tubular structure.

6. The stent of claim 1, wherein the plurality of cavities increase a flexibility of the stent (10) without substantially reducing a radial strength of the stent in a deployed state.

7. The stent of claim 1, wherein the plurality of cavities (26, 40) are micro-holes that extend from the outer surface.

8. The stent of claim 7, wherein the micro-holes have a cross section that is smaller than a cross section of a strut.

9. The stent of claim 7, wherein at least a portion of the micro-holes have geometries that are configured to provide the reservoir.

10. The stent of claim 7, wherein at least a portion of the micro-holes extend perpendicular from the outer surface to an interior of the tubular structure.

11. The stent of claim 7, wherein at least a portion of the micro-holes extend at a non-perpendicular slant angle from the outer surface to an interior of the tubular structure.

12. The stent of claim 1, wherein the plurality of cavities (26, 40) are micro-slits.

13. The stent of claim 12, wherein the micro-slits have a cross section that is smaller than a cross section of a strut.

14. The stent of claim 12, wherein at least a portion of the micro-slits have geometries that are configured to provide a reservoir for a coating substance applied to the tubular structure.

15. The stent of claim 12, wherein at least a portion of the micro-slits have a width greater than 17.78 µm (0.0007 inch).

16. The stent of claim 12, wherein at least a portion of the micro-slits have a length of at least 25.4 µm (0.001 inch).

17. The stent of claim 12, wherein at least a portion of the micro-slits extend perpendicular from the outer surface to an interior of the tubular structure.

18. The stent of claim 12, wherein at least a portion of the micro-slits extend at a non-perpendicular slant angle from the outer surface to an interior of the tubular structure.

19. The stent of claim 12, wherein at least a portion of the micro-slits have a linear geometric shape.

20. The stent of claim 12, wherein at least a portion of the micro-slits have a curved geometric shape.

21. The stent of claim 1, further comprising:
a coating substance on at least a portion of outer surface of the stent including at least a portion of the plurality of cavities (26, 40).

22. The stent of claim 21, wherein the coating substance is a restenosis inhibiting agent.

23. The stent of claim 22, wherein the restenosis inhibiting agent is selected from a drug, polymer and bio-engineered material.

24. The stent of claim 22, wherein the restenosis inhibiting agent is a combination of two agents selected from a drug, polymer and bio-engineered material.

25. The stent of claim 22, wherein each of a cavity of the plurality of cavities is configured to have a shape adapted to increase an amount of restenosis inhibiting agent coated on the stent.

26. The stent of claim 21, wherein each of a cavity of the plurality of cavities (26, 40) is configured to have a shape adapted to provide reservoir of the coated substance of the stent.

27. The stent of claim 1, comprising:
a coating substance on at least a portion of outer surface of the stent including and extending
into at least a portion of the cavities (26, 40).

28. The stent of claim 27, wherein the coating substance is on at least a portion of the inner surface of the stent.

29. The stent of claim 28, wherein each of a cavity of the plurality of cavities (26, 40) is configured to have a shape adapted to increase an amount of restenosis inhibiting agent coated on the stent (10).

30. The stent of claim 27, wherein each of a cavity of the plurality of cavities (26, 40) is configured to have a shape adapted to provide a reservoir of the coated substance on the stent (10).

31. The stent of claim 27, wherein the tubular structure is balloon expandable.

32. The stent of claim 27, wherein the tubular structure is self-expandable.

33. The stent of claim 27, wherein at least a portion of the tubular structure is made of a shape memory alloy.

34. The stent of claim 27, wherein the plurality of cavities (26, 40) are substantially evenly positioned on the tubular structure.

35. The stent of claim 27, wherein the plurality of cavities (26, 40) increase a flexibility of the stent without substantially reducing a radial strength of the stent in a deployed state.

36. The stent of claim 27, wherein the plurality of cavities (26, 40) are micro-holes.

37. The stent of claim 36, wherein the micro-holes have a cross section that is smaller than a cross section of a strut.

38. The stent of claim 27, wherein the plurality of cavities are micro-slits.

39. The stent of claim 38, wherein the micro-slits have a cross section that is smaller than a cross section of a strut.

40. The stent according to one of the preceding claims 1 to 39, wherein the cavities are distributed in the expansion struts (16) and the connector struts (18), wherein the cavities in the connector struts are of a first size and the cavities in the expansion struts are of a second size different from the first.

41. The stent of claim 1, **characterized in that** the geometry of micro-slits (40) is straight linear, curvilinear, angled or squared.

42. The stent of claim 1, **characterized in that** the connector struts are curvilinear.

43. The stent of claim 1 including micro-slits of differing patterns.

44. The stent of claim 1 including micro-slits of two different signs.

45. A method of manufacturing an intravascular stent, comprising:
forming an intravascular stent (10) having an inner surface and an outer surface, the stent having a plurality of expansion struts (16), a plurality of connector struts (18) and cells (24), wherein the width (30) of the expansion struts (16) is larger than the width (32) of the connector struts (18); and
forming a plurality of cavities (26, 40) on the outer surface of the intravascular stent,
providing as reservoirs of a coating substance, wherein the cavities extend from the outer surface of the stent through the inner surface of the stent, and wherein the cavities are oblong, square and/or oval.

46. The method of claim 45, wherein at least a portion of the plurality of cavities is formed on the outer surface of the intravascular stent (10) by a laser.

47. The method of claim 45, wherein the at least a portion of the plurality of cavities is formed on the outer surface of the intravascular stent (10) by electrical discharge machining process EDM.

48. The method of claim 45, wherein the at least a portion of the plurality of cavities (26, 40) is photochemically formed on the outer surface of the intravascular stent (10).

49. The method of claim 45 further comprising the step of applying a coating substance on the stent that inhibits restenosis.

## Patentansprüche

1. Expandierbarer Stent (10) mit:
einer Röhrenstruktur mit einer Außenfläche, die benachbart zu einer Gefäßwand positionierbar ist, einer Innenfläche, die zu einem Lumen eines Körperdurchgangs weist,
mehreren Expansionsstreben (16), Verbindungsstreben (18) und Zellen (24), wobei die Röhrenstruktur einen ersten Durchmesser, der die intraluminale Abgabe der Röhrenstruktur in den Körperdurchgang mit einem Lumen ermöglicht, sowie einen zweiten Durchmesser in der expandierten Form des Stents nach der aus dem Inneren des Röhrenteils erfolgenden Ausübung einer sich radial nach außen ausdehnenden Kraft hat,
wobei die Zellen Bereiche haben, die sich bei Expansion des Stents vergrößern, wobei die Breite (30) der Expansionsstreben (16) größer als die Breite (32) der Verbindungsstreben (18) ist, **gekennzeichnet durch**
mehrere im Stent gebildete Hohlräume (26, 40), die als Speicher für eine Beschichtungssubstanz dienen, wobei sich die Hohlräume von der Außenfläche des Stents **durch** die Innenfläche des Stents hindurch erstrecken und wobei die Hohlräume länglich, quadratisch und/oder oval sind.

2. Stent nach Anspruch 1, wobei die Röhrenstruktur mit einem Ballon expandierbar ist.

3. Stent nach Anspruch 1, wobei die Röhrenstruktur selbstexpandierbar ist.

4. Stent nach Anspruch 1, wobei zumindest ein Teil der Röhrenstruktur aus einer Formgedächtnislegierung hergestellt ist.

5. Stent nach Anspruch 1, wobei die mehreren Hohlräume (26, 40) im Wesentlichen gleichmäßig auf der Röhrenstruktur positioniert sind.

6. Stent nach Anspruch 1, wobei die mehreren Hohlräume die Flexibilität des Stents (10) erhöhen, ohne die radiale Festigkeit des Stents in einem entfalteten Zustand wesentlich zu vermindern.

7. Stent nach Anspruch 1, wobei die mehreren Hohlräume (26, 40) sich von der äußeren Oberfläche aus erstreckende Mikrolöcher sind.

8. Stent nach Anspruch 7, wobei die Mikrolöcher einen Querschnitt haben, der kleiner als der Querschnitt einer Strebe ist.

9. Stent nach Anspruch 7, wobei zumindest ein Teil der Mikrolöcher Geometrien hat, die so konfiguriert sind, dass sie den Speicher bereitstellen.

10. Stent nach Anspruch 7, wobei sich zumindest ein Teil der Mikrolöcher senkrecht von der Außenfläche zum Inneren der Röhrenstruktur erstreckt.

11. Stent nach Anspruch 7, wobei sich zumindest ein Teil der Mikrolöcher in einem nichtsenkrechten, schrägen Winkel von der Außenfläche zum Inneren der Röhrenstruktur erstreckt.

12. Stent nach Anspruch 1, wobei die mehreren Hohlräume (26, 40) Mikroschlitze sind.

13. Stent nach Anspruch 12, wobei Mikroschlitze einen Querschnitt haben, der kleiner ist, als der Querschnitt einer Strebe.

14. Stent nach Anspruch 12, wobei zumindest ein Teil der Mikroschlitze Geometrien hat, die so konfiguriert sind, dass sie einen Speicher für eine auf die Röhrenstruktur aufgetragene Beschichtungssubstanz bereitstellen.

15. Stent nach Anspruch 12, wobei zumindest ein Teil der Mikroschlitze eine Breite hat, die größer als 17,78 µm (0,0007 Inch) ist.

16. Stent nach Anspruch 12, wobei zumindest ein Teil der Mikroschlitze eine Länge von mindestens 25, 4 µm (0,001 Inch) hat.

17. Stent nach Anspruch 12, wobei sich zumindest ein Teil der Mikroschlitze senkrecht von der Außenfläche zum Inneren der Röhrenstruktur erstreckt.

18. Stent nach Anspruch 12, wobei sich zumindest ein Teil der Mikroschlitze in einem nichtsenkrechten, schrägen Winkel von der Außenfläche zum Inneren der Röhrenstruktur erstreckt.

19. Stent nach Anspruch 12, wobei zumindest ein Teil der Mikroschlitze eine lineare geometrische Form hat.

20. Stent nach Anspruch 12, wobei zumindest ein Teil der Mikroschlitze eine gekrümmte geometrische Form hat.

21. Stent nach Anspruch 1, ferner mit:
einer Beschichtungssubstanz auf mindestens einem Abschnitt der Außenfläche des Stents, der mindestens einen Anteil der mehreren Hohlräume (26, 40) aufweist.

22. Stent nach Anspruch 21, wobei die Beschichtungssubstanz ein Restenosehemmer ist.

23. Stent nach Anspruch 22, wobei der Restenosehemmer aus einem Medikament, Polymer und biotechnischem Material ausgewählt ist.

24. Stent nach Anspruch 22, wobei der Restenosehemmer eine Kombination aus zwei Wirkstoffen ist, die aus einem Medikament, Polymer und biotechnischem Material ausgewählt sind.

25. Stent nach Anspruch 22, wobei die mehreren Aushöhlungen jeweils so konfiguriert sind, dass sie eine Form haben, die geeignet ist, eine Menge von auf den Stent aufgetragenen Restenosehemmer zu vergrößern.

26. Stent nach Anspruch 21, wobei die mehreren Aushöhlungen (26, 40) jeweils so konfiguriert sind, dass sie eine Form haben, die geeignet ist, als Speicher für die Beschichtungssubstanz des Stents zu dienen.

27. Stent nach Anspruch 1, mit:
einer Beschichtungssubstanz auf mindestens einem Abschnitt der Außenfläche des Stents, der mindestens einen Anteil der Hohlräume (26, 40) umfasst und sich in mindestens einen Anteil der Hohlräume (26, 40) erstreckt.

28. Stent nach Anspruch 27, wobei die Beschichtungssubstanz auf mindestens einem Abschnitt der Innenfläche des Stents ist.

29. Stent nach Anspruch 28, wobei die mehreren Hohlräume (26, 40) jeweils so konfiguriert sind, dass sie eine Form haben, die geeignet ist, eine Menge von auf den Stent (10) aufgetragenen Restenosehemmer zu vergrößern.

30. Stent nach Anspruch 27, wobei die mehreren Aushöhlungen (26, 40) jeweils so konfiguriert sind, dass sie eine Form haben, die geeignet ist, als Speicher für die Beschichtungssubstanz auf dem Stent (10) zu dienen.

31. Stent nach Anspruch 27, wobei die Röhrenstruktur mit einem Ballon expandierbar ist.

32. Stent nach Anspruch 27, wobei die Röhrenstruktur selbstexpandierbar ist.

33. Stent nach Anspruch 27, wobei zumindest ein Teil der Röhrenstruktur aus einer Formgedächtnislegierung hergestellt ist.

34. Stent nach Anspruch 27, wobei die mehreren Hohlräume (26, 40) im Wesentlichen gleichmäßig auf der Röhrenstruktur positioniert sind.

35. Stent nach Anspruch 27, wobei die mehreren Hohlräume (26, 40) die Flexibilität des Stents erhöhen, ohne die radiale Festigkeit des Stents in einem entfalteten Zustand wesentlich zu vermindern.

36. Stent nach Anspruch 27, wobei die mehreren Hohlräume (26, 40) Mikrolöcher sind.

37. Stent nach Anspruch 36, wobei die Mikrolöcher einen Querschnitt haben, der kleiner als der Querschnitt einer Strebe ist.

38. Stent nach Anspruch 27, wobei die mehreren Hohlräume Mikroschlitze sind.

39. Stent nach Anspruch 38, wobei die Mikroschlitze einen Querschnitt haben, der kleiner als der Querschnitt einer Strebe ist.

40. Stent nach einem der vorhergehenden Ansprüche 1 bis 39, wobei die Hohlräume in den Expansionsstreben (16) und den Verbindungsstreben (18) verteilt sind, wobei die Hohlräume in den Verbindungsstreben eine erste Größe und die Hohlräume in den Expansionsstreben eine von der ersten Größe verschiedene zweite Größe haben.

41. Stent nach Anspruch 1, **dadurch gekennzeichnet, dass** die Geometrie der Mikroschlitze (40) gerade linear, kurvenförmig, winkelig oder quadratisch ist.

42. Stent nach Anspruch 1, **dadurch gekennzeichnet, dass** die Verbindungsstreben kurvenförmig sind.

43. Stent nach Anspruch 1 mit Mikroschlitzen mit verschiedenen Mustern.

44. Stent nach Anspruch 1 mit Mikroschlitzen mit zwei verschiedenen Zeichen.

45. Verfahren zur Herstellung eines intravaskulären Stents, das aufweist:
Bilden eines intravaskulären Stents (10) mit einer Innenfläche und einer Außenfläche, wobei der Stent mehrere Expansionsstreben (16), mehrere Verbindungsstreben (18) und Zellen (24) hat, wobei die Breite (30) der Expansionsstreben (16) größer als die Breite (32) der Verbindungsstreben (18) ist; und
Bilden mehrerer Hohlräume (26, 40) auf der Außenfläche des intravaskulären Stents, die als Speicher für eine Beschichtungssubstanz dienen, wobei sich die Hohlräume von der Außenfläche des Stents durch die Innenfläche des Stents hindurch erstrecken und wobei die Hohlräume länglich, quadratisch und/oder oval sind.

46. Verfahren nach Anspruch 45, wobei zumindest ein Teil der mehreren Hohlräume auf der Außenfläche des intravaskulären Stents (10) durch einen Laser gebildet ist.

47. Verfahren nach Anspruch 45, wobei der mindestens eine Teil der mehreren Hohlräume auf der Außenfläche des intravaskulären Stents (10) durch ein elektroerosives Bearbeitungsverfahren (EDM) gebildet ist.

48. Verfahren nach Anspruch 45, wobei der mindestens eine Teil der mehreren Hohlräume (26, 40) auf der Außenfläche des intravaskulären Stents (10) fotochemisch gebildet ist.

49. Verfahren nach Anspruch 45, das ferner den Schritt des Auftragens einer Restenose hemmenden Beschichtungssubstanz auf den Stent aufweist

## Revendications

1. Stent expansible (10), comprenant :
une structure tubulaire comprenant une surface externe pouvant être positionnée de manière adjacente à une paroi de vaisseau, une surface interne faisant face à une lumière d'une voie de passage corporelle, une pluralité d'entretoises d'expansion (16), d'entretoises de connecteur (18) et de cellules (24), la structure tubulaire ayant un premier diamètre qui permet la pose de manière intraluminale de la structure tubulaire dans la voie de passage corporelle ayant une lumière, et un deuxième diamètre dans la forme expansée du stent suite à l'application, à partir de l'intérieur de la structure tubulaire, d'une force s'étendant radialement vers l'extérieur, les cellules ayant des régions qui s'agrandissent suite à l'expansion du stent, dans lequel la largeur (30) des entretoises d'expansion (16) est supérieure à la largeur (32) des entretoises de connecteur (18), **caractérisé par** :
une pluralité de cavités (26, 40) formées dans le stent, servant de réservoirs d'une substance de revêtement, dans lequel les cavités s'étendent à partir de la surface externe du stent en passant par la surface interne du stent, et dans lequel les cavités sont oblongues, carrées et/ou ovales.

2. Stent selon la revendication 1, dans lequel la structure tubulaire est expansible par ballonnet.

3. Stent selon la revendication 1, dans lequel la structure tubulaire est auto-expansible.

4. Stent selon la revendication 1, dans lequel au moins une partie de la structure tubulaire est réalisée avec un alliage à mémoire de forme.

5. Stent selon la revendication 1, dans lequel la pluralité de cavités (26, 40) sont positionnées de manière sensiblement régulière sur la structure tubulaire.

6. Stent selon la revendication 1, dans lequel la pluralité de cavités augmente une flexibilité du stent (10) sans réduire sensiblement une résistance radiale du stent à l'état déployé.

7. Stent selon la revendication 1, dans lequel la pluralité de cavités (26, 40) sont des micro-trous qui s'étendent à partir de la surface externe.

8. Stent selon la revendication 7, dans lequel les micro-trous ont une section transversale qui est inférieure à une section transversale d'une entretoise.

9. Stent selon la revendication 7, dans lequel au moins une partie des micro-trous a des géométries qui sont configurées pour fournir le réservoir.

10. Stent selon la revendication 7, dans lequel au moins une partie des micro-trous s'étend perpendiculairement de la surface externe à un intérieur de la structure tubulaire.

11. Stent selon la revendication 7, dans lequel au moins une partie des micro-trous s'étend selon un angle oblique non perpendiculaire de la surface externe à un intérieur de la structure tubulaire.

12. Stent selon la revendication 1, dans lequel la pluralité de cavités (26, 40) sont des micro-fentes.

13. Stent selon la revendication 12, dans lequel les micro-fentes ont une section transversale qui est inférieure à une section transversale d'une entretoise.

14. Stent selon la revendication 12, dans lequel au moins une partie des micro-fentes a des géométries qui sont configurées pour fournir un réservoir pour une substance de revêtement appliquée sur la structure tubulaire.

15. Stent selon la revendication 12, dans lequel au moins une partie des micro-fentes a une largeur supérieure à 17,78 µm (0,0007 pouce).

16. Stent selon la revendication 12, dans lequel au moins une partie des micro-fentes a une longueur d'au moins 25,4 µm (0,001 pouce).

17. Stent selon la revendication 12, dans lequel au moins une partie des micro-fentes s'étend perpendiculairement, de la surface externe à un intérieur de la structure tubulaire.

18. Stent selon la revendication 12, dans lequel au moins une partie des micro-fentes s'étend selon un angle oblique non perpendiculaire, de la surface externe à un intérieur de la structure tubulaire.

19. Stent selon la revendication 12, dans lequel au moins une partie des micro-fentes a une forme géométrique linéaire.

20. Stent selon la revendication 12, dans lequel au moins une partie des micro-fentes a une forme géométrique incurvée.

21. Stent selon la revendication 1, comprenant en outre :
une substance de revêtement sur au moins une partie de surface externe du stent comprenant au moins une partie de la pluralité de cavités (26, 40).

22. Stent selon la revendication 21, dans lequel la substance de revêtement est un agent d'inhibition de resténose.

23. Stent selon la revendication 22, dans lequel l'agent d'inhibition de resténose est choisi parmi un médicament, un polymère et un matériau de l'ingénierie biologique.

24. Stent selon la revendication 22, dans lequel l'agent d'inhibition de resténose est une combinaison de deux agents choisis parmi un médicament, un polymère et un matériau de l'ingénierie biologique.

25. Stent selon la revendication 22, dans lequel chaque cavité de la pluralité de cavités est configurée pour avoir une forme adaptée pour augmenter une quantité d'agent d'inhibition de resténose appliquée sur le stent.

26. Stent selon la revendication 21, dans lequel chaque cavité de la pluralité de cavités (26, 40) est configurée pour avoir une forme adaptée pour fournir un réservoir de la substance de revêtement du stent.

27. Stent selon la revendication 1, comprenant :
une substance de revêtement sur au moins une partie de surface externe du stent comprenant et s'étendant dans au moins une partie des cavités (26, 40).

28. Stent selon la revendication 27, dans lequel la substance de revêtement est sur au moins une partie de la surface interne du stent.

29. Stent selon la revendication 28, dans lequel chaque cavité de la pluralité de cavités (26, 40) est configurée pour avoir une forme adaptée pour augmenter une quantité d'agent d'inhibition de resténose appliquée sur le stent (10).

30. Stent selon la revendication 27, dans lequel chaque cavité de la pluralité de cavités (26, 40) est configurée pour avoir une forme adaptée pour fournir un réservoir de la substance de revêtement sur le stent (10).

31. Stent selon la revendication 27, dans lequel la structure tubulaire est expansible par ballonnet.

32. Stent selon la revendication 27, dans lequel la structure tubulaire est auto-expansible.

33. Stent selon la revendication 27, dans lequel au moins une partie de la structure tubulaire est réalisée avec un alliage à mémoire de forme.

34. Stent selon la revendication 27, dans lequel la pluralité de cavités (26, 40) sont positionnées de manière sensiblement régulière sur la structure tubulaire.

35. Stent selon la revendication 27, dans lequel la pluralité de cavités (26, 40) augmente une flexibilité du stent sans réduire sensiblement une résistance radiale du stent dans un état déployé.

36. Stent selon la revendication 27, dans lequel la pluralité de cavités (26, 40) sont des micro-trous.

37. Stent selon la revendication 36, dans lequel les micro-trous ont une section transversale qui est inférieure à une section transversale d'une entretoise.

38. Stent selon la revendication 27, dans lequel la pluralité de cavités est des micro-fentes.

39. Stent selon la revendication 38, dans lequel les micro-fentes ont une section transversale qui est inférieure à une section transversale d'une entretoise.

40. Stent selon l'une quelconque des revendications 1 à 39 précédentes, dans lequel les cavités sont réparties dans les entretoises d'expansion (16) et les entretoises de connecteur (18), dans lequel les cavités dans les entretoises de connecteur ont une première taille et les cavités dans les entretoises d'expansion ont une deuxième taille différente de la première.

41. Stent selon la revendication 1, **caractérisé en ce que** la géométrie des micro-fentes (40) est droite, linéaire, curviligne, coudée ou carrée.

42. Stent selon la revendication 1, **caractérisé en ce que** les entretoises de connecteur sont curvilignes.

43. Stent selon la revendication 1, comprenant des micro-fentes de différents modèles.

44. Stent selon la revendication 1, comprenant des micro-fentes de deux signes différents.

45. Procédé pour fabriquer un stent intravasculaire, comprenant:
la formation d'un stent intravasculaire (10) ayant une surface interne et une surface externe, le stent ayant une pluralité d'entretoises d'expansion (16), une pluralité d'entretoises de connecteur (18) et de cellules (24), dans lequel la largeur (30) des entretoises d'expansion (16) est supérieure à la largeur (32) des entretoises de connecteur (18) ; et
la formation d'une pluralité de cavités (26, 40) sur la surface externe du stent intravasculaire, servant de réservoirs d'une substance de revêtement, dans lequel les cavités s'étendent à partir de la surface externe du stent en passant par la surface interne du stent, et dans lequel les cavités sont oblongues, carrées et/ou ovales.

46. Procédé selon la revendication 45, dans lequel au moins une partie de la pluralité de cavités est formée sur la surface externe du stent intravasculaire (10) par un laser.

47. Procédé selon la revendication 45, dans lequel la au moins une partie de la pluralité de cavités est formée sur la surface externe du stent intravasculaire (10) par un procédé d'usinage à électro-érosion EDM.

48. Procédé selon la revendication 45, dans lequel la au moins une partie de la pluralité de cavités (26, 40) est formée de manière photochimique sur la surface externe du stent intravasculaire (10).

49. Procédé selon la revendication 45, comprenant en outre l'étape consistant à appliquer une substance de revêtement sur le stent qui empêche la resténose.
